# FASCICULE DE BREVET EUROPEEN

(11) **EP 0 404 625 B1**
(45) Date de publication et mention de la délivrance du brevet: **23.10.1996**
(21) Numéro de dépôt: 90401521.1
(22) Date de dépôt: 05.06.1990
(51) Int. Cl.: C12Q 1/70, C12N 15/49

(54) **Séquences oligonucléotidiques pour l'amplification du génome des rétrovirus du type HIV-2 et SIV, et leurs applications au diagnostic in-vitro des infections dûes à ces virus**
Oligonukleotiden-Sequenzen zur Amplifizierung von HIV-2 und SIV Retrovirusgenomen, ihre Verwendung zur In-vitro-Diagnostik von durch diese Viren verursachten Infektionen
Oligonucleotide sequences for amplification of type HIV-2 and SIV retroviruses genomes and their application to in-vitro diagnostic of infections caused by these viruses

(30) Priorité: 02.06.1989 FR 8907355
(43) Date de publication de la demande: 27.12.1990
(73) Titulaire: INSTITUT PASTEUR, F-75724 Paris Cédex 15 (FR); INSTITUT NATIONAL DE LA SANTE ET DE LA RECHERCHE MEDICALE (INSERM), 75654 Paris Cédex 13 (FR)
(72) Inventeur: Sonigo, Pierre, F-75015 Paris (FR); Brechot, Christian, F-75015 paris (FR); Courgnaud, Valérie, F-75015 Paris (FR)
(74) Mandataire: Desaix, Anne

(56) Documents cités:
- EP-A- 0 229 701
- EP-A- 0 239 425
- EP-A- 0 283 327
- WO-A-86/02383
- JOURNAL OF INFECTIOUS DISEASES, vol. 158, no. 6, décembre 1988, Chicago, IL (US); M. RAYFIELD et al., pp. 1170-1176
- NATURE, vol. 328, 06 août 1987, London (GB); L. CHAKRABARTI et al., pp. 543- 547
- BIOLOGICAL ABSTRACTS DATABANK; M. RAYFIELD et al., no. 37073639
- BIOLOGICAL ABSTRACTS DATABANK; B. KORBER et al., no. 37094484
- Science vol. 239, no. 4837, 15 janvier 1988, pages 295-297, Washington, DC, US;
- BIOLOGICAL ABSTRACTS DATABANK abrégé no. 37083316; J. Courgnaud et al. page 668
- NATURE vol. 326, no. 6114, 16 avril 1987, pages 662-669, London, GB; M. Guyader et al.

## Description

La présente invention est relative à des séquences oligonucléotidiques utilisables notamment en tant qu'amorces oligonucléotidiques pour la mise en oeuvre de techniques d'amplification de séquences nucléiques de rétrovirus d'immunodéficience humaine du type HIV-2, ou de rétrovirus d'immunodéficience du singe du type SIV.

L'invention concerne en particulier l'application de ces séquences à des méthodes de diagnostic in vitro chez l'homme de l'infection d'un individu par un rétrovirus du type HIV-2.

L'isolement et la caractérisation de rétrovirus regroupés sous la désignation HIV-2 ont été décrits dans la demande de brevet EP-A-0 239 425. Ces rétrovirus ont été isolés chez plusieurs malades africains présentant des symptômes d'une lymphadénopathie ou d'un Syndrome d'Immunodeficience Acquise (SIDA).

Les rétrovirus du type HIV-2 comme les rétrovirus du type HIV-1, se caractérisent par un tropisme pour les lymphocytes T4 humains et par un effet cytopathogène à l'égard de ces lymphocytes lorsqu'ils s'y multiplient, pour alors causer soit des polyadénopathies généralisées et persistantes, soit un SIDA.

Un autre rétrovirus, dénommé SIV-1, cette dénomination remplaçant la dénomination antérieurement connue STLV-III, a été isolé chez le singe macaque rhésus (M.D. DANIEL et al. Science, 228, 1201 (1985) ; N.L. LETWIN et al, Science, 230, 71 (1985) sous l'appellation "STLV-IIImac").

Un autre rétrovirus, désigné "STLV-III_{AGM}", (ou SIV_{AGM}) a été isolé chez des singes verts sauvages. Mais contrairement aux virus présents chez le singe macaque rhésus, la présence de STLV-III_{AGM} ne semble pas induire une maladie du type SIDA chez le singe vert d'Afrique.

Pour la commodité du langage, ces virus ne seront plus désignés dans ce qui suit que par l'expression SIV (l'expression SIV est l'abréviation anglaise de "Simian Immunodeficency Virus" (Virus d'immunodéficience du singe) éventuellement suivie d'une abréviation désignant l'espèce de singe dont ils sont issus par exemple "mac" pour le macaque" ou "AGM" pour le singe vert d'Afrique (abréviation de "African Green Monkey").

Une souche du rétrovirus SIV à été déposée à la C.N.C.M le 7 février 1986 sous le n° I-521.

Des études ont montré que le rétrovirus SIV comporte certaines protéines possédant une parenté immunologique avec des protéines ou glycoprotéines susceptibles d'être obtenues dans des conditions analogues à partir de HIV-2.

La poursuite de l'étude des rétrovirus HIV-2 a également conduit à l'obtention de séquences d'ADN complémentaires (ADNc) des ARN de leur génome. La séquence nucléotidique complète d'un ADNc d'un rétrovirus représentatif de la classe HIV-2 (HIV-2 ROD) a été déposée le 21/02/1986 à la C.N.C.M. sous le n° I-532, sous le nom de référence LAV-2 ROD.

Les méthodes de diagnostic in vitro des infections par des virus du type HIV-2 existant actuellement, font le plus souvent appel à la détection d'anticorps anti-HIV-2 éventuellement présents dans un fluide biologique, notamment dans un sérum obtenu, à partir du patient à l'étude, par mise en contact de ce fluide biologique avec des extraits ou antigènes d'HIV-2, dans des conditions permettant la production d'une réaction immunologique éventuelle de ces ces extraits ou antigènes avec ces anticorps.

De telles méthodes de diagnostic sont peu sensibles et risquent d'être faussement négatives, en particulier dans le cas d'une infection récente d'un individu par le virus HIV-2.

Les techniques d'amplification génomique sont d'un appoint considérable pour la mise au point de méthodes de diagnostic in vitro particulièrement sensibles de maladies virales. Parmi ces techniques d'amplification, on peut citer la technique PCR (Polymerase Chain Reaction) telle que décrite dans les demandes de brevet EP-A-0 200 362 et EP-A-0 229 701, ou encore la technique dite "Qβreplicase" décrite dans Biotechnology, vol. 6, page 1197 (octobre 1988), et celle procédant à l'aide d'une ARN polymérase (T7 RNA polymerase) décrite dans la demande de brevet international n° WO89/01050. Elles permettent d'améliorer la sensibilité de détection des acides nucléiques des virus, mais nécessitent cependant l'utilisation d'amorces de synthèse spécifiques.

Pour la recherche des virus du type HIV-2, le choix des amorces est problématique. En effet, du fait de la grande variabilité des séquences de nucléotides du génome viral, une amorce conforme à la séquence connue d'un isolat donné d'HIV-2 peut faillir à l'amplification de certains variants viraux du type HIV-2. D'autre part, même si une amorce est choisie dans une région conservée du génome d'un virus HIV-2 à un autre, son "bon fonctionnement" n'est pas pour autant assuré et peut donner lieu à de mauvais rendements d'amplification.

Rayfield M. et al ont décrit dans une publication de "The Journal of Infections Diseases", vol. 158, n° 6, Décembre 1988, p. 1170-1176, des amorces situées dans la partie LTR de la séquence de HIV-2, pour l'amplification de l'ADN de HIV-2.

La présente invention fournit précisément des séquences (ou amorces) oligonucléotidiques permettant l'amplification à des fins diagnostiques du génome de tous virus du type HIV-2, avec de bons rendements.

Les amorces de la présente invention sont à la fois spécifiques des virus du groupe HIV-2 et des virus du type SIV, et sont insensibles aux variations du génome de ces virus.

La présente invention a pour objet des amorces oligonucléotidiques, d'environ 15 à 25 nucléotides, utilisables pour l'amplification génomique des virus du type HIV-2 ou SIV.

Une amorce selon l'invention est caractérisée en ce que sa séquence nucléotidique :
- (a) soit est choisie parmi celles qui sont contenues dans l'une des séquences nucléiques délimitées par les nucléotides situés aux positions 40 et 61, 9537 et 9558, 240 et 259, 546 et 569, 906 et 927, 612 et 633, 1857 et 1876, 2078 et 2101, 6275 et 6299, 6855 et 6878, 7548 et 7573, 7782 et 7805, 8412 et 8434, 61 et 82, 9558 et 9579, de l'ADNc dérivé du génome du virus HIV-2 ROD, ou qui sont complémentaires de l'une des séquences nucléiques sus-mentionnées,
- (b) soit est choisie parmi celles qui sont contenues dans l'une des séquences nucléiques délimitées par les nucléotides situés aux positions 40 et 61, 9511 et 9532, 240 et 259, 256 et 275, 551 et 574, 911 et 932, 617 et 638, 1868 et 1887, 2035 et 2058, 6227 et 6251, 7550 et 7564, 7776 et 7799, 8406 et 8428, 61 et 82, 9532 et 9553, de l'ADNc dérivé du génome du virus SIVmac 142, ou qui sont complémentaires de l'une des séquences nucléiques sus-mentionnées,
- (c) soit (notamment pour les amorces les plus longues) contient l'une des séquences nucléiques susdites issues de HIV-2 ROD ou de SIVmac 142, ou contient une séquence nucléique complémentaire de l'une de ces dernières séquences, étant entendu que les nucléotides supplémentaires éventuels qui "débordent" ladite séquence nucléique, de préférence du côté de l'extrémité 5', coïncident de préférence avec ceux qui se trouvent placés en deça de l'extrémité 5' correspondant au sein même de la séquence complète de HIV-2 ROD ou de SIVmac 142,
- étant également entendu que les brins des ADNc qui sont pris en considération aux points (a), (b) et (c) sont ceux qui se trouvent être complémentaires des ARN des virus HIV-2 ROD et SIVmac 142,
- (d) soit, si la séquence de cette amorce n'est pas identique à l'une des séquences nucléiques susdites, ou n'est pas complémentaire de l'une de ces séquences, néanmoins susceptible de s'hybrider avec cette séquence nucléique issue des virus HIV-2ROD et/ou SIVmac 142 sus-mentionnées dans une solution composée de Tris 10 mM, KCl 20 mM, MgCl₂ 2 mM, et 0,01 % de gélatine, pendant 1 minute à une température supérieure ou égale à 50°C.

La numérotation des nucléotides mentionnés ci-dessus correspond à celle utilisée dans la figure 2 de l'article de GUYADER et al, Nature, vol. 326, p. 662-669 (1987) pour ce qui concerne l'ADNc d'HIV-2 ROD, et à celle utilisée dans la figure 1 de l'article de CHAKRABARTI L. et al, Nature, vol. 328, p. 543-547 (1987) pour ce qui concerne l'ADNc de SIVmac 142.

L'invention concerne plus particulièrement les amorces oligonucléotidiques caractérisées par les enchaînements nucléotidiques suivants (représentés dans le sens 5' → 3' :

LTR1 GGTTCTCTCCAGCACTAGCAGG
LTR2 GGTCCTAACAGACCAGGGTC
GAG1 ATGGGCGCGAGAAACTCCGTCTTG
GAG5 ATGGTGCTGTTGGTCTACTTGT
GAG2 CCCGGCGGAAAGAAAAAGTACA
GAG2B TGTACTTTTTCTTTCCGCCGGG
POL1 TGGGGAAAGAAGCCCCGCAA
POL2 CCAAAGAGAGAATTGAGGTGCAGC
P1 CAGAAATAGGGATACTTGGGGAACC
P2 GCCTGAATAATTGGTATCATTACA
P2B TGTAATGATACCAATTATTCAGGC
P4 AGTTCTGCCACCTGTGCACTAAAGG
P6 GGGATAGTGCAGCAACAGCAACAG
P7 CATTTCCTGATCCGCCAGCTGAT
P7B ATCAGCTGGCGGATCAGGAAATG
P8 GCAGGGAACACCCAGGCTCTAC

Les amorces sus-mentionnées sont identiques ou complémentaires des séquences d'acides nucléiques suivantes, issues de l'ADNc du virus HIV-2 ROD ou de celui du virus SIVmac 142 :
- l'amorce LTR1 est identique d'une part, à la séquence nucléique comprenant les nucléotides situés aux positions 40 à 61, ainsi qu'à celle comprenant les nucléotides situés aux positions 9537 à 9558 de l'ADNc d'HIV-2 ROD, et, d'autre part, à la séquence nucléique comprenant les nucléotides situés aux positions 40 à 61, ainsi qu'à celle comprenant les nucléotides situés aux positions 9511 à 9532 de l'ADNc de SIVmac 142,
- l'amorce LTR2 est complémentaire d'une part, des séquences nucléiques comprenant les nucléotides situés aux positions 240 à 259 des ADNc de HIV-2 ROD et SIVmac 142, et, d'autre part, de la séquence nucléique comprenant les nucléotides situés aux positions 256 à 275 de l'ADNc de SIVmac 142,
- l'amorce GAG1 est identique à la séquence nucléique comprenant les nucléotides 546 à 569 de l'ADNc de HIV-2 ROD, et à la séquence nucléique comprenant les nucléotides situés aux positions 551 à 574 de l'ADNc de SIVmac 142,
- l'amorce GAG5 est complémentaire des séquences nucléiques comprenant les nucléotides situés respectivement aux positions 906 à 927 et 911 à 932 des ADNc de HIV-2 ROD et SIVmac 142,
- l'amorce GAG2 est identique aux séquences nucléiques comprenant les nucléotides situés respectivement aux positions 612 à 633 et 617 à 638 des ADNc de HIV2 ROD et SIVmac 142,
- l'amorce GAG2B est complémentaire des séquences nucléiques sus-mentionnés identiques à GAG2,
- l'amorce POL1 est identique aux séquences nucléiques comprenant les nucléotides situés respectivement aux positions 1857 à 1876, et 1868 à 1887 des ADNc de HIV-2 ROD et SIVmac 142,
- l'amorce P1 est identique aux séquences nucléiques comprenant les nucléotides situés respectivement aux positions 6275 à 6299, et 6227 à 6251 des ADNc de HIV-2 ROD et SIVmac 142,
- l'amorce P2 est complémentaire de la séquence nucléique comprenant les nucléotides situés aux positions 6855 à 6878 de l'ADNc de HIV-2 ROD,
- l'amorce P2B est complémentaire de la séquence nucléique identique à P2 sus-mentionnée,
- l'amorce P4 est complémentaire de la séquence nucléique comprenant les nucléotides situés aux positions 7548 à 7573 de l'ADNc de HIV-2 ROD, et partiellement complémentaire de la séquence nucléique comprenant les nucléotides situés aux positions 7550 à 7564 de l'ADNc de SIVmac 142,
- l'amorce P6 est identique aux séquences nucléiques comprenant les nucléotides situés respectivement aux positions 7782 à 7805, et 7776 à 7799 des ADNc de HIV-2 ROD et SIVmac 142,
- l'amorce P7 est identique aux séquences nucléiques comprenant les nucléotides situés respectivement aux positions 8412 à 8434 et 8406 à 8428 des ADNc de HIV-2 ROD et SIVmac 142,
- l'amorce P7B est complémentaire des séquences nucléiques identiques à l'amorce P7 sus-mentionnée,
- l'amorce P8 est complémentaire d'une part, à la séquence nucléique comprenant les nucléotides situés aux positions 61 à 82, ainsi qu'à celle comprenant ceux situés aux positions 9558 à 9579 de l'ADNc de HIV-2 ROD, et d'autre part, à la séquence nucléique comprenant les nucléotides situés aux positions 61 à 82, ainsi qu'à celle comprenant les nucléotides situés aux positions 9532 à 9553 de l'ADNc de SIVmac 142.

L'invention a également pour objet les amorces possédant une structure nucléotidique complémentaire de celles des amorces LTR1, LTR2, GAG1, GAG5, POL1, POL2, P1, P4, et P8 définies ci-dessus.

Elle concerne également les amorces présentant certaines mutations par rapport à celles définies ci-dessus sans que les propriétés d'hybridation, telles que définies ci-dessus, de ces amorces soient modifiées. Le pourcentage de nucléotides différents de ceux constituant les amorces décrites ci-dessus, sans pour autant affecter les propriétés d'hybridation des amorces de l'invention, est généralement compris entre 0 % et 10 %, et de préférence n'excède pas 20 %.

D'une manière générale, un plus grand nombre de mutations seront tolérées du côté 5′ que du côté 3′ de l'amorce, le côté 3′ devant s'hybrider parfaitement avec un brin déterminé d'une séquence nucléique pour permettre l'amplification de cette séquence.

L'invention s'étend également aux amorces telles que décrites ci-dessus liées au niveau de leur extrémité 5′ à un promoteur pour la mise en oeuvre d'une méthode d'amplification génomique par synthèse de multiple copies d'ARN telle que décrite dans la demande de brevet EP-A-0 310 229.

L'invention a plus particulièrement pour objet l'utilisation des amorces décrites ci-dessus pour la mise en oeuvre d'une méthode de diagnostic in vitro de l'infection d'un individu par un virus du type HIV-2.

Cette méthode de diagnostic in vitro de l'invention est réalisée à partir d'un échantillon biologique (notamment un fluide biologique tel que le sérum) obtenu à partir d'un patient à l'étude, et comprend principalement les étapes suivantes :
- une étape d'extraction de l'acide nucléique à détecter appartenant au génome du virus du type HIV-2 éventuellement présent dans l'échantillon biologique sus-mentionné, et,le cas échéant, une étape de traitement à l'aide d'une transcriptase inverse dudit acide nucléique si ce dernier est sous forme d'ARN afin d'obtenir un acide nucléique double brin,
- un cycle comprenant les étapes suivantes :
   . dénaturation de l'acide nucléique double brin à détecter , ce qui conduit à la formation d'un acide nucléique monobrin,
   . hybridation de chacun des brins d'acide nucléique, obtenus lors de l'étape de dénaturation précédente, avec au moins une amorce selon l'invention, par mise en contact des brins sus-mentionnés avec au moins un couple d'amorces selon l'invention dans les conditions d'hybridation définies ci-dessus,
   . formation, à partir des amorces, des ADN complémentaires aux brins sur lesquels elles sont hybridées (étape d'élongation) en présence d'un agent de polymérisation et de quatre nucléosides triphosphate différents, ce qui conduit à la formation d'un plus grand nombre d'acides nucléiques double brin à détecter qu'à l'étape de dénaturation précédente,
   ce cycle étant répété un nombre de fois déterminé pour obtenir ladite séquence nucléique à détecter éventuellement présente dans l'échantillon biologique dans une proportion suffisante pour permettre sa détection,
- une étape de détection de la présence éventuelle de l'acide nucléique appartenant au génome du virus du type HIV-2 dans l'échantillon biologique.

La méthode de diagnostic in vitro de l'invention peut être réalisée soit à partir de l'ARN, soit à partir de l'ADN viral.

En effet, les génomes des virus HIV-2 se présentent sous forme d'ARN ou d'ADN en fonction de la localisation du virus dans l'organisme.

Lorsque le virus est situé à l'intérieur des cellules de l'organisme, notamment à l'intérieur des cellules sanguines, son ARN est recopié en ADN par une transcriptase inverse. Par contre, le génome des virus du type HIV-2 en milieu extracellulaire, notamment dans le sang, demeure sous forme d'ARN.

L'étape d'extraction de l'ADN viral contenu dans les cellules de l'échantillon biologique est plus particulièrement détaillée dans l'article de LAURE F. et al, paru dans Lancet, p. 538-540 (1988).

A titre illustratif, les lymphocytes sont séparés des autres constituants sanguins par centrifugation dans un gradient de Ficoll. Les lymphocytes ainsi obtenus sont ensuite traités avec un tampon de lyse constitué de tris pH 8 10 mM, EDTA 10 mM, NaCl 10 mM, de 0,5 % de SDS (sodium dodecylsulfate) et de 100 µg/ml de protéinase K pendant 2 heures à 60°C. l'ADN est ensuite extrait au phenol puis précipité à l'éthanol.

L'extraction peut aussi être effectuée de façon identique à celle précédemment décrite, sur le sérum concentré. On obtient dans ce cas, de l'ARN et une étape supplémentaire de transformation de l'ARN monobrin en ADN double brin est nécessaire à effectuer lorsque le diagnostic in vitro de l'invention est réalisé à partir d'échantillons biologiques contenant les virus du type HIV-2 dont les génomes sont sous forme d'ARN.

Cette transformation de l'ARN en ADN est réalisée par traitement de l'ARN obtenu après extraction de l'échantillon biologique, notamment du sérum, dans un milieu approprié à l'aide de l'enzyme transcriptase inverse, dans les conditions indiquées par le fournisseur (Amersham par exemple).

Dans un mode de réalisation préféré de la méthode de diagnostic de l'invention, l'étape de dénaturation du cycle est réalisée pendant 1 minute à 94°C.

L'étape d'hybridation du cycle de la méthode de diagnostic in vitro de l'invention est avantageusement réalisée par mise en contact des brins d'acide nucléique obtenus lors de l'étape de dénaturation du cycle avec au moins un couple d'amorces de l'invention, ces amorces étant choisies de manière à ce que l'une de ces deux amorces s'hybride avec une séquence nucléique située sur un des deux brins tandis que l'autre s'hybride avec une séquence nucléique située sur un brin complémentaire de ce dernier, lesdites séquences nucléiques (avec lesquelles lesdites amorces sont susceptibles de s'hybrider) étant séparées, lorsque l'on considère les deux brins complémentaires sus-mentionnés regroupés dans un acide nucléique double brin, par un nombre de paire de bases compris entre 50 et 10.000, de préférence entre 100 et 2000.

L'utilisation de plusieurs couples d'amorces différents de l'invention permet l'amplification et la détection d'acides nucléiques différents du génome de HIV-2.

A titre d'exemple de couples d'amorces préférés utilisables dans le cadre de la présente invention, on peut citer les amorces LTR1 et GAG2. On peut citer également les couples P1 et P2, P2 et P7, P7 et P8, P8 et LTR2. Avantageusement, les couples d'amorces utilisés sont choisis de manière à ce que les fragments d'ADN synthétisés couvrent les régions P1 à P2, LTR1 à Pol2, P2 à P7, P7 à P8, P8 à LTR2.

L'agent de polymérisation utilisé dans l'étape d'élongation du cycle est une ADN polymérase, notamment la Taq polymérase ou encore toute polymérase appropriée pour la mise en oeuvre d'une méthode de diagnostic in vitro selon l'invention suivant le principe de la technique "QβReplicase" ou de celle décrite dans la demande de brevet international sus-mentionnée.

D'une manière générale, le cycle de la méthode de diagnostic in vitro de l'invention est répété entre 10 et 60 fois, et de préférence 40 fois.

Avantageusement, l'étape d'élongation du cycle de la méthode sus-mentionnée de l'invention est réalisée pendant 1 minute à 72°C.

A titre d'exemple pour 1 µg de l'ADN rétroviral à détecter, on utilise 10 pmoles de chaque amorce, 10 nanomoles de chaque nucléotide triphosphate (dTNP), 1 U de Taq polymérase dans un volume final de 100 µl du tampon :
Tris 10 mM pH 8.3 (mesuré à 23°C)
KCl 20 mM
MgCl₂ 2 mM
0,01 % de gélatine
et soumis 40 fois au cycle thermique suivant :
1 mn à 94°C (dénaturation)
1 mn à environ 55-60°C (hybridation)
1 mn à 72°C (élongation)

Dans un mode de réalisation préféré de la méthode de diagnostic in vitro de la présente invention, l'étape de détection de la présence éventuelle de l'acide nucléique du virus du type HIV-2 dans l'échantillon biologique est réalisée à l'aide d'une (ou plusieurs) sonde nucléotidique marquée susceptible de s'hybrider avec la (ou les) séquence d'acide nucléique amplifiée et en ce que l'on détecte les éventuels complexes d'hybridation alors formés entre la (ou les) sonde et la (ou les) séquence nucléotidique amplifiée à détecter.

L'invention a également pour objet les amorces, telles que définies ci-dessus, marquées, notamment de manière radioactive ou enzymatique, ainsi que leur utilisation en tant que sondes nucléotidiques, notamment dans le cadre de la méthode de diagnostic in vitro telle que décrite ci-dessus.

Les amorces de l'invention sont également utilisables pour la mise en oeuvre d'une méthode de diagnostic in vitro de l'infection de singes (macaque, singe de mangabeys ou singe vert) par le virus du type SIV, cette méthode reprenant les principales caractéristiques de celle décrite ci-dessus.

L'invention a également pour objet des kits de diagnostic pour la mise en oeuvre des méthodes de diagnostic in vitro sus-mentionnées. A titre d'exemple, un kit de diagnostic de la présente invention comprend :
- au moins un couple d'amorces oligonucléotidiques selon l'invention, chaque couple comprenant une amorce s'hybridant à l'un des brins de la séquence d'acide nucléique à détecter, et une amorce s'hybridant avec le brin complémentaire de ce dernier dans les conditions définies ci-dessus,
- des réactifs appropriés à la mise en oeuvre du cycle d'opérations d'amplification, notamment de l'ADN polymérase, et quatre nucléotides triphosphate différents.
- une (ou plusieurs) sonde marquée capable de s'hybrider avec la (ou les) séquence d'acide nucléique amplifiée à détecter.

L'invention concerne également un procédé de synthèse des amorces décrites ci-dessus.

L'invention a également pour objet un procédé de production d'un (ou plusieurs) peptide (ou polypeptide) comprenant :
- une étape d'amplification de la séquence nucléique codant pour ce peptide (et contenant avantageusement un promoteur pour la traduction de cette séquence) à l'aide d'un couple d'amorces selon l'invention,
- l'introduction de ladite séquence nucléique ainsi amplifiée dans un vecteur approprié,
- la transformation de cellules hôtes appropriées à l'aide du vecteur sus-mentionné,
- la mise en culture des cellules hôtes ainsi transformées et la récupération du peptide produit par ces derniers.

Les polypeptides correspondant, selon le code génétique universel, aux séquences (ou amorces) nucléotidiques décrites ci-dessus sont également utilisables en tant qu'agents immunogènes, notamment en association avec un véhicule pharmaceutiquement acceptable dans une composition pharmaceutique.

L'invention vise également un procédé de préparation des polypeptides mentionnés ci-dessus, notamment ceux correspondant selon le code génétique universel aux séquences (ou amorces) nculéotidiques décrites ci-dessus, ce procédé étant caractérisé en ce que, partant de préférence de l'aminoacide C-terminal, l'on condense successivement deux à deux les aminoacyles successifs dans l'ordre requis, ou des aminoacyles et des fragments préalablement formés et contenant déjà plusieurs résidus aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragment à l'exception des fonctions amine de l'un et carboxyle de l'autre ou vice-versa, qui doivent normalement intervenir dans la formation des liaisons peptidiques, notamment après activation de la fonction carboxyle, selon les méthodes connues dans la synthèse des peptides et ainsi de suite, de proche en proche, jusqu'à l'acide aminé N-terminal.

Par exemple, on aura recours à la technique de synthèse peptidique en solution homogène décrite par Houbenweyl dans "Methode der Organischen Chemie" (Méthode de la Chimie Organique) édité par E. Wunsch, vol. 15-I et II, THIEME, STUTTGART, 1974, ou à celle de synthèse peptidique en phase solide décrite par R.D. Merrifield dans "Solid Phase Peptide Synthesis" (J. AM. CHEM. SOC., 45, 2149-2154).

L'invention concerne également un procédé de préparation des séquences (ou amorces) nucléotidiques décrites ci-dessus, ce procédé comprenant les étapes suivantes :
- incubation de l'ADN génomique, isolé à partir d'un des virus du type HIV ou SIV sus-mentionnés, avec de l'ADNase I, puis addition d'EDTA et purification par extraction au mélange phenol/chloroforme/alcool isoamylique (25/24/ 1) puis par l'éther,
- traitement de l'ADN ainsi extrait par de l'Eco R1 méthylase en présence de DTT, et purification par extraction telle que décrite ci-dessus,
- incubation de l'ADN ainsi purifié avec les 4 désoxynucléotides triphosphates dATP, dCTP, dGTP, et dTTP en présence de T4 ADN polymérase et d'ADN ligase de E. coli, puis purification selon la méthode décrite ci-dessus,
- le clonage des acides nucléiques ainsi obtenus dans un vecteur approprié et la récupération de l'acide nucléique recherché à l'aide d'une sonde appropriée.

Un procédé de préparation particulièrement avantageux des séquences nucléotidiques de l'invention comprend les étapes suivantes :
- la synthèse d'ADN en utilisant la méthode automatisée des β-cyanethyl phosphoramidite décrite dans Bioorganic Chemistry 4; 274-325 (1986),
- le clonage des acides nucléiques ainsi obtenus dans un vecteur approprié et la récupération de l'acide nucléique par hybridation avec une sonde appropriée.

Un autre procédé de préparation des séquences nucléotidiques de l'invention comprend les étapes suivantes :
- l'assemblage d'oligonucléotides synthétisés chimiquement, pourvus à leurs extrémités de sites de restriction différents, dont les séquences sont compatibles avec l'enchaînement en acides aminés du polypeptide naturel selon le principe décrit dans Proc. Natl. Acad. Sci. USA, 80; 7461-7465, (1983),
- le clonage des acides nucléiques ainsi obtenus dans un vecteur approprié et la récupération de l'acide nucléique recherché par hybridation avec une sonde appropriée.

## Revendications

1. Amorce oligonucléotidique, d'environ 15 à 25 nucléotides, utilisable pour l'amplification génomique des virus HIV-2 ou SIVmac caractérisée en ce que sa séquence nucléotidique :
- (a) soit est choisie parmi celles qui sont contenues dans l'une des séquences nucléiques délimitées par les nucléotides situés aux positions 40 et 61, 9537 et 9558, 240 et 259, 546 et 569, 906 et 927, 612 et 633, 1857 et 1876, 2078 et 2101, 6275 et 6299, 6855 et 6878, 7548 et 7573, 7782 et 7805, 8412 et 8434, 61 et 82, 9558 et 9579, de l'ADNc dérivé du génome du virus HIV-2 ROD, ou qui sont complémentaires de l'une des séquences nucléiques sus-mentionnées,
- (b) soit est choisie parmi celles qui sont contenues dans l'une des séquences nucléiques délimitées par les nucléotides situés aux positions 40 et 61, 9511 et 9532, 240 et 259, 256 et 275, 551 et 574, 911 et 932, 617 et 638, 1868 et 1887, 2035 et 2058, 6227 et 6251, 7550 et 7564, 7776 et 7799, 8406 et 8428, 61 et 82, 9532 et 9553, de l'ADNc dérivé du génome du virus SIVmac 142, ou qui sont complémentaires de l'une des séquences nucléiques sus-mentionnées,
- (c) soit (notamment pour les amorces les plus longues) contient l'une des séquences nucléiques susdites issues de HIV-2 ROD ou de SIVmac 142, ou contient une séquence nucléique complémentaire de l'une de ces dernières séquences, étant entendu que les nucléotides. supplémentaires éventuels qui "débordent" ladite séquence nucléique, de préférence du côté de l'extrémité 5', coïncident de préférence avec ceux qui se trouvent placés en deça de l'extrémité 5' correspondant au sein même de la séquence complète de HIV-2 ROD ou de SIVmac 142,
- étant également entendu que les brins des ADNc qui sont pris en considération aux points (a), (b) et (c) sont ceux qui se trouvent être complémentaires des ARN des virus HIV-2 ROD et SIVmac 142,
- (d) soit, si la séquence de cette amorce n'est pas identique à l'une des séquences nucléiques susdites, ou n'est pas complémentaire de l'une de ces séquences, néanmoins susceptible de s'hybrider avec cette séquence nucléique issue des virus HIV-2ROD et/ou SIVmac 142 sus-mentionnées dans une solution composée de Tris 10 mM, KCl 20 mM, MgCl₂, 2mM, et 0,01 % de gélatine, pendant 1 minute une température supérieure ou égale à 50°C.

2. Amorces oligonucléotidiques selon la revendication 1, caractérisées par les enchaînements nucléotidiques suivants : 
LTR1 | GGTTCTCTCCAGCACTAGCAGG
LTR2 | GGTCCTAACAGACCAGGGTC
GAG1 | ATGGGCGCGAGAAACTCCGTCTTG
GAG5 | ATGGTGCTGTTGGTCTACTTGT
GAG2 | CCCGGCGGAAAGAAAAAGTACA
GAG2B | TGTACTTTTTCTTTCCGCCGGG
POL1 | TGGGGAAAGAAGCCCCGCAA
POL2 | CCAAAGAGAGAATTGAGGTGCAGC
P1 | CAGAAATAGGGATACTTGGGGAACC
P2 | GCCTGAATAATTGGTATCATTACA
P2B | TGTAATGATACCAATTATTCAGGC
P4 | AGTTCTGCCACCTGTGCACTAAAGG
P6 | GGGATAGTGCAGCAACAGCAACAG
P7 | CATTTCCTGATCCGCCAGCTGAT
P7B | ATCAGCTGGCGGATCAGGAAATG
P8 | GCAGGGAACACCCAGGCTCTAC
 ou leurs séquences complémentaires.

3. Amorces oligonucléotidiques selon la revendication 2, présentant des mutations, sans que soient pour autant modifiées les propriétés d'hybridation telles que définies dans la revendication 1 de ces amorces.

4. Méthode de diagnostic in vitro de l'infection d'un individu par un virus du type HIV-2, cette méthode étant réalisée à partir d'un échantillon biologique, notamment d'un sérum, prélevé chez cet individu, et comprenant principalement les étapes suivantes :
- une étape d'extraction de l'acide nucléique à détecter appartenant au génome du virus du type HIV-2 éventuellement présent dans l'échantillon biologique sus-mentionné, et, le cas échéant, une étape de traitement à l'aide d'une transcriptase inverse dudit acide nucléique si ce dernier est sous forme d'ARN afin d'obtenir un acide nucléique double brin,
- un cycle comprenant les étapes suivantes :
. dénaturation de l'acide nucléique double brin à détecter, notamment par traitement de l'acide nucléique pendant 1 minute à 94°C, ce qui conduit à la formation d'acide nucléique monobrin,
. hybridation de chacun des brins d'acide nucléique obtenus lors de l'étape de dénaturation, précédente avec au moins une amorce selon l'une quelconque des revendication 1 à 3, par mise en contact des brins sus-mentionnés avec au moins un couple d'amorces selon l'une des revendications 1 a 3 dans les conditions d'hybridation définies dans la revendication 1,
. élongation des amorces le long des brins sur lesquels elles sont hybridées en présence d'un agent de polymérisation, tel que la Taq polymérase, et de quatre nucléosides triphosphate différents, ce qui conduit à la formation d'un plus grand nombre d'acide nucléique double brin à détecter qu'à l'étape de dénaturation précédente,
ce cycle étant répété de préférence entre 10 et 60 fois,
- une étape de détection de la présence éventuelle de l'acide nucléique appartenant au génome du virus du type HIV-2 dans l'échantillon biologique.

5. Méthode de diagnostic selon la revendication 4, caractérisée en ce que l'étape d'hybridation du cycle est réalisée par mise en contact des brins d'acide nucléique obtenus lors de l'étape de dénaturation précédente, avec au moins un couple d'amorces selon l'une des revendications 1 à 3, ces amorces étant choisies de manière à ce que l'une de ces deux amorces s'hybride avec une séquence nucléique située sur un des deux brins tandis que l'autre s'hybride avec une séquence nucléique située sur un brin complémentaire de ce dernier, lesdites séquences nucléiques (avec lesquelles lesdites amorces sont susceptibles de s'hybrider) étant séparées, lorsque l'on considère les deux brins complémentaires sus-mentionnés regroupés dans un acide nucléique double brin, par un nombre de paire de bases compris entre 50 et 10000, de préférence entre 100 et 2000.

6. Méthode selon la revendication 5, caractérisée en ce que les couples d'amorces utilisés sont tels que les fragments d'acide nucléique synthétisés couvrent les régions LTR1 à Pol2, P1 à P2, P2 à P7, P7 à P8, P8 à LTR2.

7. Méthode selon l'une des revendications 4 à 6, caractérisée en ce que ce que l'étape de détection de la présence éventuelle de l'acide nucléique du virus du type HIV-2 dans l'échantillon biologique est réalisée à l'aide d'une (ou plusieurs) sonde nucléotidique marquée susceptible de s'hybrider avec la (ou les) séquence nucléique amplifiée et en ce que l'on détecte les éventuels complexes d'hybridation alors formés entre la (ou les) sonde et la (ou les) séquence nucléotidique amplifiée à détecter.

8. Kit de diagnostic pour la mise en oeuvre d'une méthode selon l'une des revendications 4 à 7, caractérisé en ce que qu'il comprend
- au moins un couple d'amorces selon l'une quelconque des revendications 1 à 3,
- des réactifs appropriés à la mise en oeuvre du cycle d'amplification, notamment de l'ADN polymérase, et quatre nucléotides triphosphate différents.
- une (ou plusieurs) sonde marquée capable de s'hybrider avec la (ou les) séquence d'acide nucléique amplifiée à détecter.

9. Méthode de préparation d'un polypeptide déterminé comprenant :
- une étape d'amplification de la séquence nucléique codant ledit polypeptide à l'aide d'un couple d'amorces selon l'une des revendications 1 à 3,
- l'introduction de ladite séquence nucléique ainsi amplifiée dans un vecteur approprié,
- la transformation de cellules hôtes à l'aide du vecteur sus-mentionné,
- la mise en culture des cellules hôtes transformées par ledit vecteur et la récupération du polypeptide produit par ces derniers.

10. Procédé de préparation des séquences (ou amorces) nucléotidiques selon l'une quelconque des revendications 1 à 3, comprenant les étapes suivantes :
- incubation de l'ADN génomique, isolé à partir d'un des virus HIV-2 ROD et SIVmac 142, avec de l'ADNase I, puis addition d'EDTA et purification par extraction au mélange phenol/chloroforme/alcool isoamylique (25/24/ 1) puis par l'éther,
- traitement de l'ADN ainsi extrait par de l'Eco R1 méthylase en présence de DTT, et purification par extraction au mélange phenol/chloroforme/alcool isoamylique (25/24/ 1) puis par l'éther,
- incubation de l'ADN ainsi purifié avec les 4 désoxynucléotides triphosphates dATP, dCTP, dGTP, et dTTP en présence de T4 ADN polymerase et d'ADN ligase de E. coli, puis purification par extraction au mélange phénol/chloroforme/alcool isoamylique (25/24/1) puis par l'éther, et
- clonage des acides nucléiques ainsi obtenus dans un vecteur approprié et la récupération de l'acide nucléique recherché à l'aide d'une sonde appropriée.

## Patentansprüche

1. Oligonudeotidprimer mit ungefähr 15 bis 25 Nucleotiden, der für die Genomamplifikation des Virus HIV-2 oder SlVmac einsetzbar ist, dadurch gekennzeichnet, daß seine Nucleotidsequenz:
- (a) entweder aus denjenigen ausgewählt ist, die in einer der Nucleotidsequenzen enthalten sind, die von den Nucleotiden begrenzt sind, die an den Positionen 40 und 61, 9537 und 9558, 240 und 259, 546 und 569, 906 und 927, 612 und 633, 1857 und 1876, 2078 und 2101, 6275 und 6299, 6855 und 6878, 7548 und 7573, 7782 und 7805, 8412 und 8434, 61 und 82, 9558 und 9579 der vom Genom des Virus HIV-2 ROD abgeleiteten cDNA liegen, oder die komplementär zu einer der oben genannten Nucleotidsequenzen sind,
- (b) oder aus denjenigen ausgewählt ist, die in einer der Nucleotidsequenzen enthalten sind, die von den Nucleotiden begrenzt sind, die an den Positionen 40 und 61, 9511 und 9532, 240 und 259, 256 und 275, 551 und 574, 911 und 932, 617 und 638, 1868 und 1887, 2035 und 2058, 6227 und 6251, 7550 und 7564, 7776 und 7799, 8406 und 8428, 61 und 82, 9532 und 9553 der vom Genom des Virus SlVmac 142 abgeleiteten cDNA liegen, oder die komplementär zu einer der eben genannten Nucleotidsequenzen sind,
- (c) oder (insbesondere für die längsten Primer) eine der oben genannten Nucleotidsequenzen enthält, die aus HIV-2 ROD oder SlVmac 142 stammen, oder eine Nucleotidsequenz enthält, die komplementär zu einer der letzteren Sequenzen ist, wobei sich versteht, daß eventuelle zusätzliche Nucleotide, die vorzugsweise von der Seite des 5'-Endes über besagte Nucleotidsequenz "hinausragen", vorzugsweise mit denjenigen übereinstimmen, die sich diesseits des entsprechenden 5'-Endes innerhalb der vollständigen Sequenz des HIV-2 ROD oder des SlVmac 142 befinden,
- wobei sich ebenso versteht, daß sich die cDNA-Stränge, die unter den Punkten (a), (b) und (c) in Betracht gezogen werden, diejenigen sind, die sich als komplementär zur RNA der Viren HIV-2 ROD und SlVmac 142 erweisen,
- (d) oder, falls die Sequenz dieses Primers nicht identisch mit einer der oben genannten Nucleotidsequenzen oder nicht komplementär zu einer dieser Sequenzen ist, nichtsdestoweniger geeignet ist, mit dieser Nucleotidsequenz, die aus den oben genannten Viren HIV-2 ROD und/oder SlVmac 142 stammt, in einer Lösung, die sich aus 10 mM Tris, 20 mM KCI, 2 mM MgCl₂ und 0,01 % Gelatine zusammensetzt, während 1 Minute bei einer Temperatur, die größer oder gleich 50°C ist, zu hybridisieren.

2. Oligonucleotidprimer nach Anspruch 1, gekennzeichnet durch folgende Nucleotidsequenzen: 
LTR1 | GGTTCTCTCCAGCACTAGCAGG
LTR2 | GGTCCTAACAGACCAGGGTC
GAG1 | ATGGGCGCGAGAAACTCCGTCTTG
GAG5 | ATGGTGCTGTTGGTCTACTTGT
GAG2 | CCCGGCGGAAAGAAAAAGTACA
GAG2B | TGTACTTTTTCTTTCCGCCGGG
POL1 | TGGGGAAAGAAGCCCCGCAA
POL2 | CCAAAGAGAGAATTGAGGTGCAGC
P1 | CAGAAATAGGGATACTTGGGGAACC
P2 | GCCTGAATAATTGGTATCATTACA
P2B | TGTAATGATACCAATTATTCAGGC
P4 | AGTTCTGCCACCTGTGCACTAAAGG
P6 | GGGATAGTGCAGCAACAGCAACAG
P7 | CATTTCCTGATCCGCCAGCTGAT
P7B | ATCAGCTGGCGGATCAGGAAATG
P8 | GCAGGGAACACCCAGGCTCTAC
 oder deren Komplementärsequenzen.

3. Oligonucleotidprimer nach Anspruch 2, die Mutationen aufweisen, ohne daß jedoch die Hybridisierungseigenschaften dieser Primer, wie sie in Anspruch 1 definiert sind, verändert sind.

4. Verfahren zur in vitro-Diagnose der Infektion eines Individuums mit einem Virus des Typs HIV-2, wobei diese Methode ausgehend von biologischen Probe, insbesondere einem Serum, die diesem Individuum entnommen wurde, realisiert wird und im wesentlichen folgende Schritte umfaßt:
- einen Schritt, bei dem die zu detektierende Nucleinsäure, die zu dem Genom des Virus des Typs HIV-2 gehört und die möglicherweise in der oben genannten biologischen Probe vorhanden ist, extrahiert wird und
- ggf. einen Schritt, bei dem mit Hilfe einer reversen Transkriptase besagte Nucleinsäure behandelt wird, wenn letztere in Form einer RNA vorliegt, um eine doppelsträngige Nucleinsäure zu erhalten,
- einen Zyklus, der folgende Schritte umfaßt:
- Denaturierung der zu erfassenden, doppelsträngigen Nucleinsäure, insbesondere durch Behandlung der Nucleinsäure während 1 Minute bei 94°C, was zur Bildung einzelsträngiger Nucleinsäure führt,
- Hybridisierung jedes der Nucleinsäurestränge, die während des vorangegangenen Denaturierungsschritts erhalten wurden, mit wenigstens einen der Primer gemäß einem der Ansprüche 1 bis 3, wobei die Stränge mit wenigstens einem Primerpaar gemäß einem der Ansprüche 1 bis 3 unter den Hybridisierungsbedingungen, die in Anspruch 1 definiert sind, in Kontakt gebracht werden,
- Verlängerung der Primer entlang der Stränge, an denen sie hybridisiert sind, unter Anwesenheit eines Polymerisationswirkstoffs, wie der Taq-Polymerase, und von vier unterschiedlichen Nucleosidtriphosphaten, was zur Bildung einer größeren Anzahl der zu ermittelnden doppelsträngigen Nucleinsäure führt, als bei dem vorangegangenen Denaturierungsschritt,
wobei dieser Zyklus vorzugsweise 10- bis 60mal wiederholt wird,
- ein Schritt, bei dem das mögliche Vorhandensein von Nucleinsäure, die zum Genom des Virus des Typs HIV-2 gehört, in der biologischen Probe detektiert wird.

5. Diagnoseverfahren nach Anspruch 4, dadurch gekennzeichnet, daß der Hybridisierungsschritt des Zyklus dadurch realisiert wird, daß die während des vorangegangenen Denaturierungsschrittes erhaltenen Nucleinsäurestränge mit wenigstens einem Paar von Primern nach einem der Ansprüche 1 bis 3 in Kontakt gebracht werden, wobei diese Primer so ausgesucht sind, daß einer dieser beiden Primer mit einer Nucleotidsequenz hybridisiert, die auf einem der beiden Stränge angeordnet ist, während der andere mit einer Nucleotidsequenz hybridisiert, die auf einem zu letzterem komplementären Strang angeordnet ist, wobei die Nucleotidsequenzen (mit denen die Primer hybridisieren können), wenn man die beiden komplementären Stränge wieder als in einer doppelsträngigen Nucleinsäure angeordnet betrachtet, durch eine Anzahl von Basenpaaren getrennt sind, die zwischen 50 und 10.000, vorzugsweise zwischen 100 und 2.000 liegt.

6. Verfahren nach Anspruch 5, dadurch gekennzeichnet, daß die verwendeten Primerpaare wie die Nucleinsäurefragmente synthetisiert sind und die Bereiche LTR1 bis Pol2, P1 bis P2, P2 bis P7, P7 bis P8, P8 bis LTR2 abdecken.

7. Verfahren nach einem der Ansprüche 4 bis 6, dadurch gekennzeichnet, daß der Schritt, bei dem das mögliche Vorhandensein von Nucleinsäuren des Virus des Typs HIV-2 in der biologischen Probe ermittelt wird, mit Hilfe einer (oder mehreren) markierten Nucleotidsonde realisiert wird, die in der Lage ist, mit der (oder den) amplifizierten Nucleotidsequenz zu hybridisieren, wobei man die möglichen Hybridisierungskomplexe, die sich zwischen der (oder den) Sonde und der (oder den) zu erfassenden, amplifizierten Nucleotidsequenz gebildet haben, detektiert.

8. Diagnosekit zur Durchführung eines Verfahrens nach einem der Ansprüche 4 bis 7, dadurch gekennzeichnet, daß er aufweist:
- wenigstens ein Paar von Primern nach einem der Ansprüche 1 bis 3,
- Reagenzien, die zur Durchführung des Amplifikationszyklusses geeignet sind, insbesondere DNA-Polymerase und vier unterschiedliche Nucleosidtriphosphate,
- eine (oder mehrere) markierte Sonde, die in der Lage ist, mit der (oder den) zu erfassenden, amplifizierten Nucleotidsequenz zu hybridisieren.

9. Verfahren zur Herstellung eines bestimmten Polypeptids, das folgende Schritte umfaßt:
- einen Schritt, bei dem die Nucleotidsequenz, die das Polypeptid codiert, mit Hilfe eines Paars von Primern nach einem der Ansprüche 1 bis 3 amplifiziert wird,
- Einbringen der so amplifizierten Nucleotidsequenz in einen geeigneten Vektor,
- Transformation von Wirtszellen mit Hilfe des Vektors,
- Anlegen einer Kultur der durch den Vektor transformierten Wirtszellen und Gewinnung des Polypeptids, das durch letztere produziert wird.

10. Verfahren zur Herstellung von Nucleotidsequenzen (oder Primern) nach einem der Ansprüche 1 bis 3, das die folgenden Schritte umfaßt:
- Inkubation der Genom-DNA, die aus einem der Viren HIV-2 ROD und SlVmac 142 isoliert wurde, mit DNAase 1, nachfolgendes Hinzufügen von EDTA und Reinigung durch Extraktion mit einer Mischung aus Phenol/Chloroform/lsoamylalkohol (25/24/1), anschließend durch Ether,
- Behandlung der so extrahierten DNA mit Eco R1 Methylase unter Anwesenheit von DTT und Reinigung durch Extraktion mit einer Mischung aus Phenol/Chloroform/lsoamylalkohol (25/24/1), anschließend durch Ether,
- Inkubation der so gereinigten DNA mit den vier Desoxynucleotidtriphospaten dATP, dCTP, dGTP und dTTP unter Anwesenheit von T4 DNA-Polymerase und DNA-Ligase von E. coli, dann Reinigung durch Extraktion mit einer Mischung aus Phenol/Chloroform/lsoamylalkohol (25/24/1), anschließend durch Ether, und
- Klonen der so erhaltenen Nucleinsäuren in einem geeigneten Vektor und Gewinnung der gesuchten Nucleinsäure mit Hilfe einer geeigneten Sonde.

## Claims

1. Oligonucleotide primer of approximately 15 to 25 nucleotides which can be used for the genomic amplification of the HIV-2 or SIVmac viruses, characterized in that its nucleotide sequence:
- (a) is either selected from among those which are contained in one of the nucleic acid sequences which are delimited by the nucleotides situated in positions 40 and 61, 9537 and 9558, 240 and 259, 546 and 569, 906 and 927, 612 and 633, 1857 and 1876, 2078 and 2101, 6275 and 6299, 6855 and 6878, 7548 and 7573, 7782 and 7805, 8412 and 8434, 61 and 82, and 9558 and 9579, of the cDNA which is derived from the genome of the virus HIV-2 ROD, or which are complementary to one of the abovementioned nucleic acid sequences,
- (b) or is selected from among those which are contained in one of the nucleic acid sequences which are delimited by the nucleotides situated in positions 40 and 61, 9511 and 9532, 240 and 259, 256 and 275, 551 and 574, 911 and 932, 617 and 638, 1868 and 1887, 2035 and 2058, 6227 and 6251, 7550 and 7564, 7776 and 7799, 8406 and 8428, 61 and 82, and 9532 and 9553, of the cDNA which is derived from the genome of the virus SIVmac 142, or which are complementary to one of the above-mentioned nucleic acid sequences,
- (c) or (particularly for the longest primers) contains one of the abovementioned nucleic acid sequences derived from HIV-2 ROD or from SIVmac 142, or contains a nucleic acid sequence which is complementary to one of these latter sequences, it being understood that the possible additional nucleotides which "extend beyond" the said nucleic acid sequence, preferably in the direction of the 5' end, preferably coincide with those which are located on the near side of the corresponding 5' end within the complete sequence of HIV-2 ROD or SIVmac 142,
- it also being understood that the strands of the cDNAs which are taken into consideration in items (a), (b) and (c) are those which are found to be complementary to the RNAs of the HIV-2 ROD and SIVmac 142 viruses.
- (d) or, if the sequence of this primer is not identical to one of the abovementioned nucleic acid sequences, or is not complementary to one of these sequences, is nevertheless capable of hybridizing with this nucleic acid sequence which is derived from the abovementioned HIV-2ROD and/or SIVmac 142 viruses in a solution consisting of 10 mM Tris, 20 mM KCl, 2 mM MgCl₂ and 0.01% gelatin, over the space of 1 minute and at a temperature greater than or equal to 50°C.

2. Oligonucleotide primers according to Claim 1, characterized by the following nucleotide sequences: 
LTR1 | GGTTCTCTCCAGCACTAGCAGG
LTR2 | GGTCCTAACAGACCAGGGTC
GAG1 | ATGGGCGCGAGAAACTCCGTCTTG
GAG5 | ATGGTGCTGTTGGTCTACTTGT
GAG2 | CCCGGCGGAAAGAAAAAGTACA
GAG2B | TGTACTTTTTCTTTCCGCCGGG
POL1 | TGGGGAAAGAAGCCCCGCAA
POL2 | CCAAAGAGAGAATTGAGGTGCAGC
P1 | CAGAAATAGGGATACTTGGGGAACC
P2 | GCCTGAATAATTGGTATCATTACA
P2B | TGTAATGATACCAATTATTCAGGC
P4 | AGTTCTGCCACCTGTGCACTAAAGG
 
P6 GGGATAGTGCAGCAACAGCAACAG
P7 CATTTCCTGATCCGCCAGCTGAT
P7B ATCAGCTGGCGGATCAGGAAATG
P8 GCAGGGAACACCCAGGCTCTAC
 or their complementary sequences.

3. Oligonucleotide primers according to Claim 2, which exhibit mutations without, for all that, the hybridization properties, such as defined in Claim 1, of these primers being modified.

4. Method for the in-vitro diagnosis of the infection of an individual with a virus of the HIV-2 type, this method being carried out using a biological sample, in particular a serum, which has been removed from this individual, and principally comprising the following steps:
- a step for extracting the nucleic acid which is to be detected and which belongs to the genome of the virus of the HIV-2 type which may be present in the abovementioned biological sample and, if need be, a step for treating, with the aid of reverse transcriptase, the said nucleic acid if this latter is in the form of RNA in order to obtain a double-stranded nucleic acid,
- a cycle comprising the following steps:
. denaturation of the double-stranded nucleic acid to be detected, in particular by treatment of the nucleic acid for 1 minute at 94°C, thereby resulting in the formation of single-stranded nucleic acid,
. hybridization of each of the nucleic acid strands which are obtained in the preceding denaturation step with at least one primer according to any one of Claims 1 to 3 by bringing the abovementioned strands into contact with at least one pair of primers according to one of Claims 1 to 3 under the hybridization conditions defined in Claim 1,
. elongation of the primers along the whole length of the strands to which they are hybridized in the presence of a polymerization agent such as Taq polymerase and four different nucleoside triphosphates, thereby resulting in the formation of a larger double-stranded nucleic acid number to be detected than at the preceding denaturation step,
this cycle preferably being repeated between 10 and 60 times, and
- a step for detecting the possible presence of the nucleic acid belonging to the genome of the virus of the HIV-2 type in the biological sample.

5. Diagnostic method according to Claim 4, characterized in that the hybridization step of the cycle is carried out by bringing the nucleic acid strands which have been obtained in the preceding denaturation step into contact with at least one pair of primers according to one of Claims 1 to 3, these primers being selected such that one of these two primers hybridizes with a nucleic acid sequence which is situated on one of the two strands while the other hybridizes with a nucleic acid sequence which is situated on a strand which is complementary to the latter, the said nucleic acid sequences (with which the said primers are capable of hybridizing) being separated, when the two abovementioned complementary strands are considered as being united in one double-stranded nucleic acid, by a number of base pairs which is between 50 and 10,000, preferably between 100 and 2000.

6. Method according to Claim 5, characterized in that the pairs of primers which are used are such that the nucleic acid fragments which are synthesized cover the regions LTR1 to Pol2, P1 to P2, P2 to P7, P7 to P8 and P8 to LTR2.

7. Method according to one of Claims 4 to 6, characterized in that the step for detecting the possible presence of the nucleic acid of the virus of the HIV-2 type in the biological sample is carried out with the aid of a (or several) labelled nucleotide probe(s) which is/are capable of hybridizing with the amplified nucleic acid sequence(s), and in that the possible hybridization complexes which are then formed between the probe(s) and the amplified nucleotide sequence(s) to be detected are detected.

8. Diagnostic kit for implementing a method according to one of claims 4 to 7, characterized in that it comprises
- at least one pair of primers according to any one of Claims 1 to 3,
- appropriate reagents for implementing the amplification cycle, in particular DNA polymerase and four different nucleotide triphosphates,
- a (or several) labelled probe(s) which is/are capable of hybridizing with the amplified nucleic acid sequence(s) to be detected.

9. Method for preparing a given polypeptide, comprising:
- a step for amplifying, with the aid of a pair of primers according to one of Claims 1 to 3, the nucleic acid sequence encoding said polypeptide,
- introduction of the said nucleic acid sequence which has thus been amplified into an appropriate vector,
- transformation of host cells with the abovementioned vector,
- culturing the host cells which have been transformed with the said vector and recovery of the polypeptide produced by the latter.

10. Process for preparing the nucleotide sequences (or primers) according to any one of Claims 1 to 3, comprising the following steps:
- incubation of the genomic DNA, which has been isolated from one of the viruses HIV-2 ROD and SIVmac 142, with DNAase I, followed by addition of EDTA and purification by means of extraction with the mixture phenol/chloroform/isoamyl alcohol (25/24/1) and then with ether,
- treatment of the DNA which has thus been extracted with Eco R1 methylase in the presence of DTT, and purification by means of extraction with the mixture phenol/chloroform/isoamyl alcohol (25/24/1) and then with ether,
- incubation of the DNA which has thus been purified with the 4 deoxynucleotide triphosphates dATP, dCTP, dGTP and dTTP in the presence of T4 DNA polymerase and E. coli DNA ligase, followed by purification by means of extraction with the mixture phenol/chloroform/isoamyl alcohol (25/24/1) and then with ether, and
- cloning of the nucleic acids which have thus been obtained in an appropriate vector and recovery of the sought-after nucleic acid with the aid of an appropriate probe.
